# EUROPEAN PATENT APPLICATION

(11) **EP 4 292 581 A1**
(43) Date of publication of application: **20.12.2023**
(21) Application number: 22382579.5
(22) Date of filing: 16.06.2022
(51) Int. Cl.: A61K 8/64, A61Q 5/10

(54) **HAIR REPIGMENTING COMPOSITION**

(71) Applicant: Bella Aurora Labs, S.A., 08690 Santa Coloma de Cervelló (Barcelona) (ES); Bordignon, Matteo, 35126 Padova (IT)
(72) Inventor: Bordignon, Matteo, Padova (IT); Hernández Navarro, Sergi, Santa Coloma de Cervelló (ES); Segura Tejedor, Jordi, Santa Coloma de Cervelló (ES)
(74) Representative: Pons

(57) **Abstract**

The present invention refers to the cosmetic use of a MIA (melanoma inhibitory activity) protein inhibitor peptide as hair repigmenting agent. It refers also to a method for hair repigmenting using a MIA peptide inhibitor. Said peptide shows an increase of the expression of *IRF4* gene, and that the combination of said peptide and commercially available peptide derivatives shows a synergistic effect on the expression of *IRF4* and/or *TYR* genes. *IRF4* gene is a biomarker associated with hair greying and *TYR* gene is a key enzyme in melanin synthesis.

## Description

### Field of the invention

The present invention relates to the field of compositions for restoration of original hair pigmentation.

### Background art

The appearance of hair plays an important role in people's overall physical appearance and self-perception. With today's increasing life expectation, the desire to look youthful plays a bigger role than ever. The hair care industry has become aware of this and also more capable to deliver active products that are directed toward meeting this consumer demand. Topical anti-aging compounds for hair include humefactants, hair conditioners, photoprotectors, and antioxidants, as disclosed in R. M. Trüeb, Aging of hair, J. Cosmet. Dermatol., 2005, 4(2), 60-72.

In mammals, the coloration of the skin and of the hair is due to a common category of pigments: melanins. These melanins are produced in the skin by melanocytes, cells which are located at the level of the basement membrane of the epidermis and in the hair follicles. Melanin synthesis is controlled by the activity of at least three enzymes: tyrosinase, DOPAchrome tautomerase (TRP-2, for Tyrosinase Related Protein 2) and DHICAoxidase (TRP-1, for Tyrosinase Related Protein 1).

The progressive loss of hair pigmentation with age is called canities or senile "hair greying". The "hair greying" is a consequence of melanogenesis reduction during the anagen phase of the hair cycle and seems to be universal. The anagen phase is the active growth phase of hair follicles. As disclosed in De Tollenaere *et al.,* Global Repigmentation Strategy of Grey Hair Follicles by Targeting Oxidative Stress and Stem Cells Protection, Appl. Sci, 2021, 11, 1533, it has been evidenced that in the case of canities, the melanocytes inside the pigmentary unit of the hair bulb lose their dendritic aspect to become more rounded and thus less differentiated and less melanogenically active, and that another hypothesis suggests that melanosome formation and their transfer could be defective.

In the art, different compounds and combination of compounds have been proposed for the repigmentation of the hair: levodopa (EP-A-0565010); retinoid compound comprising a phenolic or naphtholic functional group (US5942531); a combination of vitamin A, vitamin C, vitamin E, zinc and selenium (US-A-2002/0034484); a combination of vitamin B12, copper, folic acid and vitamin C (WO-A-2004/075849); N-acetylphosphatidyethanolamines (EP-A-1609461); lipoic acid, oltipraz, kahweol, cafestol, angelicalactone, diallyl sulfide, benzyl isothiocyanate and mixtures thereof (WO-A-2006/070101); ellagic acid (FR-A-2903324); coumarin and derivatives thereof, butylated hydroxyanisole, ethoxyquine and mixtures thereof (US-A-2008/0026018); curcumin (FR-A-2902321); sulphur, panthenol, a mixture of essential oils, bismuth salt and oxidation activator (FR-A-2904216); bisphosphonate derivative (US-A-2011/0105435); dihydroquercetin and an amino acid (US-A-2012/0114583); tyrosine and/or tyrosine derivative, protein hydrolyzate, adenosine 5'-triphosphate,riboflavin or lyxoflavin, and acetyl methionyl arginine ethyl ester (DE-A-102008047945); carnitine and dihydroquercetin (WO-A-2011/009761); purine and/or a purine derivative and a oligonucleotide (WO-A-2011/009758);

Another approach to maintain the pigmentation of the hair is based on the administration of an effective amount of at least one active agent inducing the expression of DOPAchrome tautomerase to protect melanocytes of hair follicles, as disclosed in US-A-2005/0208086.

Another approach found in the art is the use of peptides for repigmenting the hair.

In US-A-2005/0187164 it is disclosed a series of hexapeptide derivatives, which reproduce the peripheral effects of α-melanocyte stimulating hormone (MSH). The binding of said hormone to its receptor melanocortin-1 stimulates the production of tyrosinase.

In WO-A-2005/116068 it is disclosed a series of peptides and peptide conjugate derivatives of MSH, containing 3 to 6 amino acids and including the sequence d-Phe-Arg, which have a substantial activating effect on melanogenesis in the melanocytes of the hair bulb, and which are suitable to be used in the cosmetic treatment of canities or to help prevent greying hair.

In WO-A-2014/080376 it is disclosed a series of proline based di- and tripeptides with pro-pigmenting activity acting mainly on the stimulation of melanin synthesis.

In US-A-2017/0252281 it is disclosed a series of oligopeptides comprising three units of glutamic acid, which stimulates the melanogenesis for preventing and/or reducing the graying of the hair.

Thus, there is still a need to provide an effective repigmenting agent for hair.

### Subject-matter of the invention

The subject-matter of the present invention is the cosmetic use of a MIA inhibitor as hair repigmenting agent.

Another aspect of the invention is a method for hair repigmenting using a MIA inhibitor.

### Figures

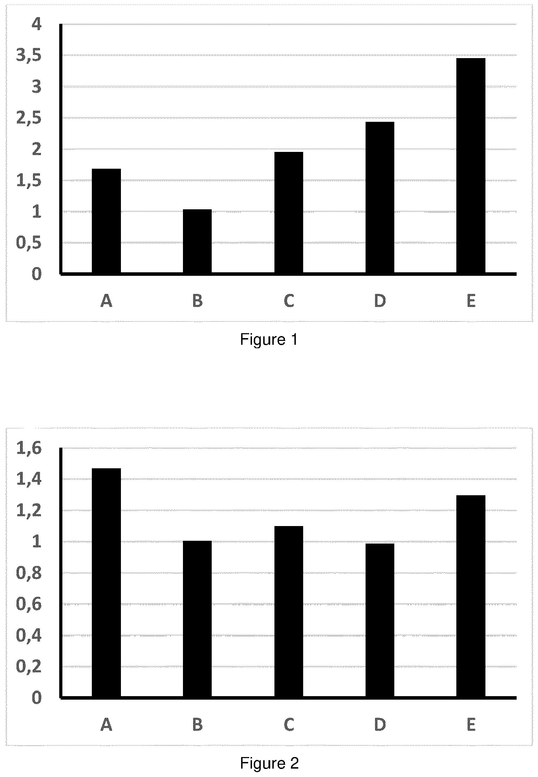

Figure 1 shows the results of gene expression by qPCR for *IRF4* gene. In ordinates the *IRF4* gene relative expression is represented. In abscises, column A is the Control group without the treatment with the inhibitor compound FH535, column B is the Control group treated with compound FH535, column C is the group treated with compound FH535 and the MIA inhibitor peptide at 0.001% of a solution comprising 1000 ppm (final concentration of MIA inhibitor 0.01 ppm), column D is the group treated with compound FH535 and palmitoyl prolyl proline dipeptide at 0.001% of a solution comprising 6000 ppm (final concentration of palmitoyl prolyl proline dipeptide 0.06 ppm), and column E is the group treated with compound FH535 and the combination of the MIA inhibitor peptide and palmitoyl prolyl proline dipeptide at 0.001% of a solution comprising 500 ppm of the former and 3000 ppm of the latter (final concentration of MIA inhibitor 0.005 ppm and palmitoyl prolyl proline dipeptide 0.03 ppm). It is observed that 0.01 ppm of MIA inhibitor peptide increase the expression of *IRF4* gene, and that the combination of 0.005 ppm of MIA inhibitor peptide and 0.03 ppm of palmitoyl prolyl proline dipeptide shows a synergistic effect on the expression of *IRF4* gene, because the observed increase for the combination is higher than the increase foreseen for the addition of the individual contributions.
Figure 2 shows the results of gene expression by qPCR for *TYR* gene. In ordinates the *TYR* gene relative expression is represented. In abscises, column A is the Control group without the treatment with the inhibitor compound FH535, column B is the Control group treated with compound FH535, column C is the group treated with compound FH535 and the MIA inhibitor peptide at 0.001% of a solution comprising 1000 ppm (final concentration of MIA inhibitor 0.01 ppm), column D is the group treated with compound FH535 and palmitoyl prolyl proline dipeptide at 0.001% of a solution comprising 6000 ppm (final concentration of palmitoyl prolyl proline dipeptide 0.06 ppm), and column E is the group treated with compound FH535 and the combination of the MIA inhibitor peptide and palmitoyl prolyl proline dipeptide at 0.001% of a solution comprising 500 ppm of the former and 3000 ppm of the latter (final concentration of MIA inhibitor 0.005 ppm and palmitoyl prolyl proline dipeptide 0,03ppm). It is observed a synergistic effect of the combination of 0.005 ppm of MIA inhibitor peptide and 0.03 ppm of palmitoyl prolyl proline dipeptide on the expression of *TYR* gene, because the observed increase for the combination is higher than the increase foreseen for the addition of the individual contributions.

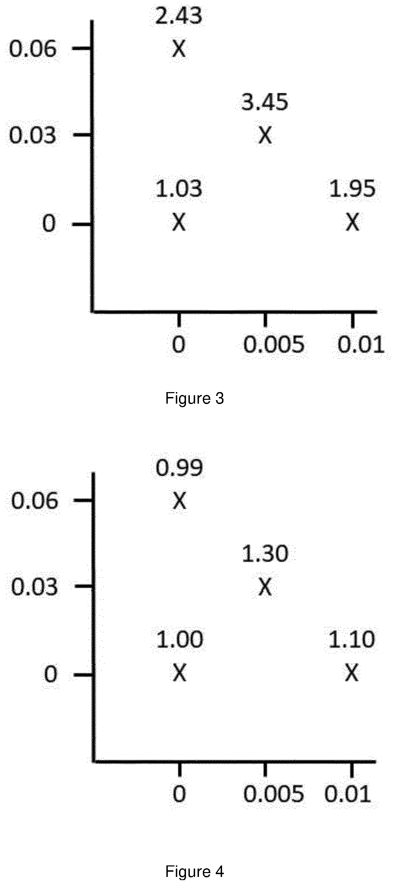

Figure 3 shows the results of gene expression by qPCR for *IRF4* gene. The concentration of palmitoyl prolyl proline dipeptide in ppm is represented in ordinates and the concentration of MIA inhibitor peptide in ppm is represented in abscises. It is observed that 0.01 ppm of MIA inhibitor peptide increase the expression of *IRF4* gene, and that the combination of 0.005 ppm of MIA inhibitor peptide and 0.03 ppm of palmitoyl prolyl proline dipeptide shows a synergistic effect on the expression of *IRF4* gene, because the observed increase for the combination is higher than the increase foreseen for the addition of the individual contributions.
Figure 4 shows the results of gene expression by qPCR for *TYR* gene. The concentration of palmitoyl prolyl proline dipeptide in ppm is represented in ordinates and the concentration of MIA inhibitor peptide in ppm is represented in abscises. It is observed a synergistic effect of the combination of 0.005 ppm of MIA inhibitor peptide and 0.03 ppm of palmitoyl prolyl proline dipeptide on the expression of *TYR* gene, because the observed increase for the combination is higher than the increase foreseen for the addition of the individual contributions.

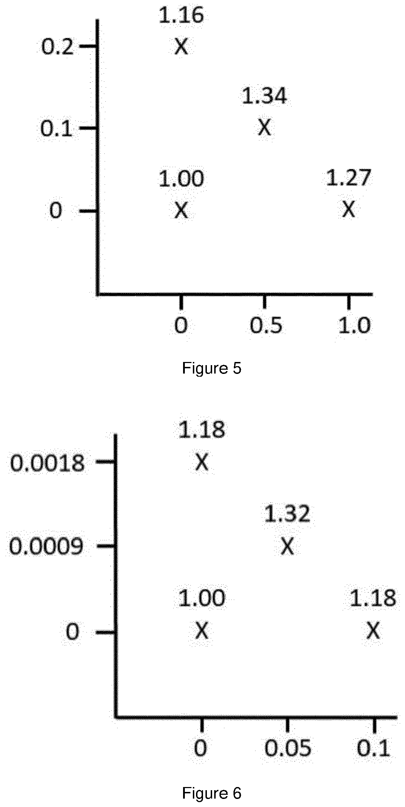

Figure 5 shows the results of gene expression by qPCR for *TYR* gene. The concentration of peptide SEQ ID NO: 50 in ppm is represented in ordinates and the concentration of MIA inhibitor peptide in ppm is represented in abscises. It is observed a synergistic effect of the combination of 0.5 ppm of MIA inhibitor peptide and 0.1 ppm of peptide SEQ ID NO: 50 on the expression of *TYR* gene, because the observed increase for the combination is higher than the increase foreseen for the addition of the individual contributions.
Figure 6 shows the results of gene expression by qPCR for *TYR* gene. The concentration of peptide SEQ ID NO: 51 in ppm is represented in ordinates and the concentration of MIA inhibitor peptide in ppm is represented in abscises. It is observed a synergistic effect of the combination of 0.05 ppm of MIA inhibitor peptide and 0.0009 ppm of peptide SEQ ID NO: 51 on the expression of *TYR* gene, because the observed increase for the combination is higher than the increase foreseen for the addition of the individual contributions.

### Detailed description of the invention

The object of the present invention is the cosmetic use of a MIA inhibitor peptide as hair repigmenting agent, wherein the MIA inhibitor peptide has a sequence selected from SEQ ID NO: 1 to SEQ ID NO: 49, or a derivative thereof.

The inventors of the present invention have developed a new use of a MIA inhibitor peptide for the repigmentation of hair. In an *in vitro* test, surprisingly, said peptide shows an increase of the expression of *IRF4* gene, and that the combination of said peptide and commercially available peptide derivatives (PP dipeptide, peptide defined by SEQ ID NO:50 and peptide defined by SEQ ID NO:51) shows a synergistic effect on the expression of *IRF4* and/or *TYR* genes. As disclosed in Adhikari et al., A genome-wide association scan in admixed Latin American identifies loci influencing facial and scalp hair features, Nature, 2016, 7:10815, *IRF4* gene is a biomarker associated with hair greying and *TYR* gene is a key enzyme in melanin synthesis.

In the present description, as well as in the claims, the singular forms "a", "an" and "the" include the plural reference unless the context clearly indicates otherwise. The term "about" refers to a deviation of plus/minus 10%, preferably plus/minus 5%. The percentages are expressed in % by weight (wt%), unless stated the contrary. The ranges defined by the terms "between ... and ..." or by the terms "from ...to..." are meant to include also said stated endpoints thereof, and they also include any narrower sub-range.

The amino acids are represented herein using the one-letter code, as is well-known by the skilled in the art, according to IUPAC-IUB Commission on Biochemical Nomenclature, as described in the book Biochemical Nomenclature and Related Documents, 2nd edition, Portland Press, 1992, London [ISBN 1-85578-005-4]. Following said nomenclature, the peptides of this description are represented with the N-terminus on the left side of the formula and with the C-terminus on the right side of the formula.

In the present description, D aminoacids are represented in lowercase, bold and underlined letters.

### MIA inhibitor peptide

The MIA inhibitor peptide of the present invention is a substance having activity as inhibitor of the human melanoma inhibitory activity (MIA) protein, in particular, those disclosed in the international patent application WO-A-2012/127352, namely, a peptide having a sequence selected from SEQ ID NO: 1 to SEQ ID NO: 49:
- SEQ ID NO: 01 VPHIPPN
- SEQ ID NO: 02 MPPTQVS
- SEQ ID NO: 03 QMHPWPP
- SEQ ID NO: 04 QPPFWQF
- SEQ ID NO: 05 TPPQGLA
- SEQ ID NO: 06 IPPYNTL
- SEQ ID NO: 07 AVRPAPL
- SEQ ID NO: 08 GAKPHPQ
- SEQ ID NO: 09 QQLSPLP
- SEQ ID NO: 10 GPPPSPV
- SEQ ID NO: 11 LPLTPLP
- SEQ ID NO: 12 QLNVNHQARADQ
- SEQ ID NO: 13 TSASTRPELHYP
- SEQ ID NO: 14 TFLPHQMHPWPP
- SEQ ID NO: 15 VPHIPPNSMALT
- SEQ ID NO: 16 RLTLLVLIMPAP
- SEQ ID NO: 17 YNLPKVSSNLSP
- SEQ ID NO: 18 MPPTQVSKFRLI
- SEQ ID NO: 19 ANIDATPLFLRA
- SEQ ID NO: 20 LLRTTETLPMFL
- SEQ ID NO: 21 SALEPLV
- SEQ ID NO: 22 GSPTPNA
- SEQ ID NO: 23 APSHATH
- SEQ ID NO: 24 TTVGHSD
- SEQ ID NO: 25 THFSTFT
- SEQ ID NO: 26 SLLLDTS
- SEQ ID NO: 27 SVAMKAHKPLLP
- SEQ ID NO: 28 NTIPGFASKSLD
- SEQ ID NO: 29 VSNYKFYSTTSS
- SEQ ID NO: 30 VSRHQSWHPHDL
- SEQ ID NO: 31 HLNILSTLWKYR
- SEQ ID NO: 32 HNASPSWGSPVM
- SEQ ID NO: 33 SHPWNAQRELSV
- SEQ ID NO: 34 HHWPFWRTLPLS
- SEQ ID NO: 35 WHTKFLPRYLPS
- SEQ ID NO: 36 NNTSFTVVPSVP
- SEQ ID NO: 37 SHLSTWKWWQNR
- SEQ ID NO: 38 FHWHPRLWPLPS
- SEQ ID NO: 39 WHWTYGWRPPAM
- SEQ ID NO: 40 FHWRYPLPLPGQ
- SEQ ID NO: 41 WHWPLFIPNTTA
- SEQ ID NO: 42 WHNGIWWHYGVR
- SEQ ID NO: 43 HHLNYLWPWTRV
- SEQ ID NO: 44 FWHRWSTFPEQP
- SEQ ID NO: 45 WHMSYFWTRPPQ
- SEQ ID NO: 46 FHLNWPSRADYL
- SEQ ID NO: 47 WHKNTNWPWRTL
- SEQ ID NO: 48 ALSPSQSHPVRS
- SEQ ID NO: 49 GTQSTAIPAPTD
or a derivative thereof.

In an embodiment, the MIA inhibitor peptide has a sequence selected from SEQ ID NOs 12-20 and 27-49, or a derivative thereof; preferably selected from SEQ ID NOs 34, 37, 39-43 and 45-47, or a derivative thereof.

In an embodiment, the MIA inhibitor peptide has a sequence selected from SEQ ID NOs 34, 38, 40 and 41, or a derivative thereof.

In an embodiment, the MIA inhibitor peptide has the sequence SEQ ID NO: 40, or a derivative thereof.

In a preferred embodiment, the MIA inhibitor peptide has the sequence SEQ ID NO: 40.

A derivative of a peptide having a sequence selected from SEQ ID NO: 1 to SEQ ID NO: 49 means a peptide with some small variations around these sequences, still retaining the MIA inhibitory activity. Examples of suitable variations are described below.

One suitable derivative is a peptide having one of the above sequences wherein one additional amino acid is present or wherein one amino acid of the sequence is deleted.

Another suitable derivative is a peptide wherein one or more of the amino acids of the sequence is/are substituted by another natural or non-natural amino acid, preferably no more than 3 amino acids of the sequence are substituted, preferably no more than 2 amino acids, and more preferably only one amino acid is substituted by another natural or non-natural amino acid.

Natural amino acids, as is well-known in the art, are those 20 amino acids which usually occur in natural proteins and peptides.

Also as is well-known in the art, non-natural (or unnatural or unusual) amino acids are based on natural amino acids but wherein one or more atoms are substituted with functional groups comprising up to 50 atoms selected from C, H, N, S, O, P, F, Cl, Br, I and Se. Suitable, non-limiting, examples of such non-naturally occurring amino acids are trans-3-methylproline, 2,4-methanoproline, cis-4-hydroxyproline, trans-4-hydroxyproline, *N*-methylglycine, allo-threonine, *N*-methylthreonine, hydroxyethylcysteine, hydroxyethylhomocysteine, nitroglutamine, homoglutamine, pipecolic acid, tert-leucine, norvaline, 2-azaphenylalanine, 3-azaphenylalanine, 4-azaphenylalanine, and 4-fluorophenylalanine. Several methods are known in the art for incorporating non-naturally occurring amino acid residues into peptides or proteins.

Other suitable derivatives are those peptides of sequences 1 to 49, also including those having amino acid additions/deletions or substitutions, as discussed above, wherein at least one amino acid is modified, i.e., an additional moiety or functional group is added thereto. Such modifications may have the purpose of improving the stability, solubility and/or skin penetration of the peptide. Suitable modifications can be selected from glycosylation, acetylation (e.g., N-terminus), amidation (e.g., C-terminus), hydroxylation (e.g., hydroxyproline), carboxylation (e.g., γ-carboxyglutamate), phosphorylation, alkylation, N-terminus lipidation (i.e., adding saturated or unsaturated C12-C18 fatty acids, such as myristic or palmitic, at the N-terminus, through an amide bond), or prenylation. Common modifications of the peptide sequences are acetylation of the N-terminus and amidation of the C-terminus. C-terminal amidation includes the formation of terminal amido groups of formula -CONH₂, -CONHR or -CONR₂, wherein R is typically a short (e.g., C₁-C₄) alkyl group. The simplest and most common C-terminal amido group is -CO-NH₂.

In a one embodiment, the MIA inhibitor peptide derivative is a peptide which is acetylated at the N-terminus and/or amidated at the C-terminus, preferably is acetylated at the N-terminus and amidated at the C-terminus.

In a particularly preferred embodiment, the MIA inhibitor is a peptide:
- selected from SEQ ID NO: 1 to SEQ ID NO: 49, preferably selected SEQ ID NOs 12-20 and 27-49, more preferably selected from SEQ ID NOs 34, 37, 39-43 and 45-47 or from SEQ ID NOs 34, 38, 40 and 41, and still more preferably is a peptide of SEQ ID NO: 40; and wherein optionally
- at least one amino acid of the sequence is modified and wherein the modification is selected from glycosylation, acetylation, amidation (C-terminal), hydroxylation (hydroxyproline), carboxylation (gamma-carboxyglutamate), phosphorylation, alkylation, N-terminal lipidation and prenylation, preferably the sequence is acetylated at the N-terminus and/or amidated at the C-terminus, and more preferably is acetylated at the N-terminus and amidated at the C-terminus.

The term "MIA inhibitor peptide" or "MIA inhibitor", as used interchangeably herein, refer widely to the MIA inhibitor substance used in the composition, either if it is strictly a peptide sequence or a modified sequence as disclosed above.

In an embodiment, the MIA inhibitor peptide has a sequence as defined above and is not modified.

Those peptides and derivatives thereof can be prepared following standard methods in peptide synthesis, typically, by solid phase synthesis, or may be obtained commercially from several sources.

The composition of the invention may comprise one or more MIA inhibitor peptide, i.e., a single MIA inhibitor substance or the combination of two or more MIA inhibitors. When more than one MIA inhibitor is used, they may be used in any proportion and, therefore, the amount of MIA inhibitor as stated herein means the total amount of MIA inhibitor substances contained in the composition.

As disclosed in the international patent application WO-A-2012/127352 or in the international patent application WO-A-03/064457 or in the article Stoll et al., The extracellular human melanoma inhibitory activity (MIA) protein adopts an SH3 domain-like fold, EMBO J., 2001, 20(3), 340-349, the activity as MIA inhibitors of the peptides of sequences 1 to 49 was determined in a phage display screening assay using recombinant human MIA.

The derivatives of the peptides of sequences 1 to 49, as discussed above, can be tested for their MIA inhibitory activity using standard methods, for example, a ligand binding assay using recombinant human MIA, as discussed in Stoll *et al., op. cit.,* or as disclosed in Schmidt et al., Targeting melanoma metastasis and immunosuppression with a new mode of melanoma inhibitory activity (MIA) protein inhibition, 2012, PLoS ONE 7(5): e37941.

The MIA inhibitor peptide shows *in vitro* activity even in low ppm content (0.01 ppm) as shown in the examples.

Said peptide shows an increase of the expression of *IRF4* gene, and the combination of said peptide and commercially available peptide derivatives shows a synergistic effect on the expression of *IRF4* and/or *TYR* genes. Taking into account that IRF4 gene is a biomarker associated with hair greying and *TYR* gene is a key enzyme in melanin synthesis, by using said peptide or combination of peptides, it is possible both to avoid or reduce the greying of the hair and to achieve repigmentation, that is to reduce or resolve the greying of the hair.

### Combination of peptides

In an embodiment of the use the MIA inhibitor peptide of the present invention as hair repigmenting agent, the MIA inhibitor peptide is combined with another peptide selected from peptide PP (Pro Pro), SEQ ID NO: 50 (LAH**f**RW) and SEQ ID NO: 51 (HfRW), wherein in:
- SEQ ID NO: 50, the first aminoacid residue is norleucine (Nle), and the fourth residue is d-Phe, and
- SEQ ID NO: 51, the second residue is d-Phe

The N-terminus of the peptide PP may include a palmitoyl group. In a preferred embodiment, the N-terminus of the peptide PP includes a palmitoyl group.

The N-terminus of SEQ ID NO: 50 may include an acetyl group, and the C-terminus an amide group (-NH₂). In a preferred embodiment, the N-terminus of SEQ ID NO: 50 includes an acetyl group, and the C-terminus an amide group (-NH₂).

The N-terminus of SEQ ID NO: 51 may include a palmitoyl group and the C-terminus an amide group (-NH₂). In a preferred embodiment, the N-terminus of SEQ ID NO: 51 includes a palmitoyl group and the C-terminus an amide group (-NH₂).

In an embodiment, the MIA inhibitor peptide is combined with peptide PP.

In an embodiment, the MIA inhibitor peptide is combined with peptide of SEQ ID NO: 50.

In an embodiment, the MIA inhibitor peptide is combined with peptide of SEQ ID NO: 51.

The combination of the MIA inhibitor peptide and peptide PP produces, surprisingly, a synergistic effect on the expression of *IRF4* and *TYR* genes in an *in vitro* test, as shown in the Examples section, and in Figures 3 and 4.

The combination of the MIA inhibitor peptide and a peptide selected from the group consisting of SEQ ID NO:50 and SEQ ID NO:51 produces, surprisingly, a synergistic effect on the expression of *TYR* gene in an *in vitro* test, as shown in the Examples section, and in Figures 5 and 6.

### Compositions

In an embodiment the MIA inhibitor peptide is comprised in a composition, which comprises at least a cosmetically acceptable excipient.

The term "excipient" or "cosmetically acceptable excipient" means a component of a cosmetic composition that is not an active ingredient, which is useful for preparing the cosmetic composition and is generally safe and non-toxic for human cosmetic use. In particular, the composition of the invention is typically intended to be used externally, topically applied over the face, body, neck, scalp, hair, eyelashes, or body hair, so the cosmetically acceptable excipient is specifically "dermatologically acceptable", i.e., it is suitable and non-toxic for use in contact with human skin tissue.

In an embodiment, the composition according to present invention is for topical administration, i.e., it is to be spread over the face, body, neck, scalp, hair, eyelashes, or body hair. Topical, dermal, or cutaneous administration are used herein interchangeably.

Any type of formulation suitable for topical administration may be used. Typically, the composition of the invention is in the form of cream, gel, cremigel, lotion, paste, foam, solution, suspension, emulsion, milk, or stick preparation, for example, which are well-known in the art. In an embodiment, the composition is in the form of a lotion.

A cremigel, for example, is a type of formulation halfway between a cream and a gel: unlike a cream, a cremigel is a gelled aqueous solution or a gelled oil, but it looks opaque as a cream, usually white in colour.

Depending the concentration of the MIA inhibitor peptide and the cosmetically acceptable excipient, the composition constitutes, for example, a concentrated active ingredient, or a less concentrated final composition directly for the user.

In an embodiment, the cosmetically acceptable excipient used according to the invention include a cosmetic carrier, preferably aqueous, alcoholic or hydroalcoholic.

In an embodiment, the composition is in the form of a lotion and the lotion comprises a cosmetic carrier selected from aqueous, alcoholic or hydroalcoholic.

In an embodiment, a suitable carrier may be, for example, anhydrous, as mixtures of fats, waxes, animal and plant oils and solid and liquid hydrocarbons.

Emulsions may be, typically, oil-in-water emulsions, water-in-oil emulsions, water-in-oil-in-water, oil-in-water-in-oil or water-in-silicone emulsions. An emulsion may generally be described as having a continuous aqueous phase (oil-in-water and water-in-oil-in-water) or a continuous oil phase (water-in-oil and oil-in-water-in-oil). The oil phase may comprise silicone oils, non-silicone oils such as paraffin hydrocarbons, fatty alcohols (e.g., stearyl alcohol, cetyl alcohol or cetostearyl alcohol), fatty acids (e.g., stearic acid, oleic acid), fatty acid esters (e.g., isopropyl myristate, isopropyl palmitate), waxes or plant oils (e.g., castor oil, canola oil, cottonseed oil, jojoba oil or arachis oil), or mixtures thereof. The aqueous phase may comprise water or a water solution of hydrophilic substances, such as alcohols (e.g., ethanol, isopropyl alcohol), polyols (e.g., glycerol, sorbitol), alpha hydroxy acids, amino acids, protein hydrolysates, simple sugars, or polysaccharides.

Emulsifiers, which are common components of emulsions, are surface-active agents (surfactants) and include non-ionic surfactants, anionic surfactants, cationic surfactants and amphoteric surfactants. Non-ionic surfactants include, among others, ethoxylated fatty alcohols, ethoxylated fatty acid esters, alkyl glucosides or alkyl oligoglucosides, ethoxylated sorbitan fatty acid esters, monoglycerol/polyglycerol fatty acid esters, ethoxylated glycerin monesters, ethoxylated polyglyceryl esters, alkyl dimethylamine oxides, or poloxamers, among others. Anionic surfactants include alkaline soaps, alkyl sulphates, alkyl ether sulphates, alkyl sulphosuccinates, alkyl phosphates, acyl sarcosinates or acyl isethionates, among others. Cationic surfactants include quaternary ammonium salts, or pyridine salts, among others. Amphoteric surfactants include imidazoline derivatives, betaines, amidobetaines and sulphobetaines.

Other common ingredients in the formulation may be selected from emollients, humectants, penetration enhancers, preservatives, viscosity controlling agents, antioxidants, pH regulators, UV filters, chelating agents, vitamins, anti-dandruff agents, perfumes, colorants, plant extracts, skin care active ingredients, hair care active ingredients, and mixtures thereof. Said additional ingredients of the compositions and the procedures for preparing them are disclosed, for example, in the CTFA International cosmetic ingredient dictionary & handbook (13th Ed. 2010) (published by the Cosmetic, Toiletry, and Fragrance Combination, Inc., Washington, D.C), or in Remington The Science and Practice of Pharmacy, 20th Edition, A. R. Gennaro, Ed., Mack Publishing Co., Lippincott Williams & Wilkins, Philadelphia, 2000, or in Cosmetic Formulation. Principles and Practice, Benson H.A.E., Roberts M.S., Rodrigues Leite-Silva V. and Walters K.A., editors, CRC Press, 2019. Also, regulated cosmetic substances and ingredients are disclosed in the European Commission database "Coslng" (https://ec.europa.eu/growth/tools-databases/cosing).

Common emollients are, for example, paraffin hydrocarbons, silicones, fatty alcohols, fatty acids, esters of fatty acids with alcohols, triglycerides, ceramides, phospholipids and waxes. Humectants include polyhydroxy alcohols, proteins and hydroxyl acids. Common preservatives include sorbic acid and its salts, benzoic acid and its salts, parabens, imidazolidinyl urea, diazolidinyl urea, DMDM hydantoin, sodium hydroxymethylglycinate, methylchloroisothiazolinone/methylisothiazolinone, benzyl alcohol and 2-phenoxyethanol, among others. Other additives may also be added for controlling the viscosity of the formulation, for example, xanthan gum, gellan gum, acacia, carrageenans, chitosan, collagen, tragacanth, pectin, starch derivatives, carbomers, cellulose derivatives (hydroxyethylcellulose, hydroxypropylcellulose, hydroxypropyl methylcellulose, methylcellulose, ethylcellulose, or carboxymethylcellulose, for example), polyamides, glutamides, colloidal silica or waxes (e.g., beeswax or vegetable waxes), among others.

Some ingredients of the composition may act as penetration enhancers, to facilitate the penetration of the active ingredients throughout the stratum corneum. Common penetration enhancers are, for example, short chain alcohols such as ethanol or isopropyl alcohol; long chain alcohols such as decanol, hexanol, lauryl alcohol, myristyl alcohol, octanol, octyldodecanol or oleyl alcohol; cyclic amides such as azone; esters such as ethyl acetate octyl salicylate, padimate O, ethyl oleate, glyceryl monoleate, glyceryl monocaprate, glyceryl tricaprylate, isopropyl myristate, isopropyl palmitate, propylene glycol monolaurate, or propylene glycol monocaprylate; ether alcohols such as Transcutol^{®}; fatty acids such as lauric acid, linoleic acid, linolenic acid, myristic acid, oleic acid, palmitic acid, stearic acid or isostearic acid; glycols such as dipropylene glycol, propylene glycol, 1,2-butylene glycol or 1,3- butylene glycol; pyrrolidones such as *N*-methyl-2-pyrrolidone or 2-pyrrolidone; sulphoxides such as decylmethyl sulphoxide or dimethyl sulphoxide; anionic surfactants; cationic surfactants; non-ionic surfactants; terpenes such as eugenol D-limonene, menthol, menthone, farnesol or neridol (as disclosed, for example, in Lane M.E., Skin penetration enhancers, Int. J. Pharm., 2013, 447, 12-21; or in Haque et al., Chemical enhancer: a simplistic way to modulate barrier function of the stratum corneum, Adv. Pharm. Bull., 2018, 8(2), 169-179).

In an embodiment, the composition comprises a penetration enhancer.

The composition of the invention has typically a slightly acidic pH, close to the physiological pH of the skin. Common acidity regulators are organic acids, including hydroxy acids and fatty acids. Some of the most common pH regulators in cosmetic emulsions are hydroxy acids such as lactic or citric acid.

One excipient can perform more than one function. The above cited ingredients, as well as many others suitable dermatological formulation excipients, are well-known to the skilled formulator of topical compositions. Such ingredients are commercially available from several companies, such as Comercial Química Massó, SA, Evonik, DuPont or Dow Corning, among others.

The preparation of the topical composition is made according to procedures well-known to the skilled in the formulation of dermatological compositions, generally involving simple steps of mixing, and optionally heating the component ingredients.

A cream, for example, can be typically prepared by warming the oily phase and the aqueous phase separately to a temperature of about 60 to 80 ºC, and then mixing under stirring to prepare the emulsion.

Typically, for example, a cream, gel, or lotion base formulation is first prepared, using standard ingredients and procedures well-known in the art, and then the MIA inhibitor peptide (either in free form or encapsulated) are added and thoroughly mixed, and the pH of the composition is finally adjusted.

In a cosmetic composition according to the invention, the MIA inhibitor peptide to be present in an effective amount, are generally present in an amount ranging between 1×10⁻⁷ wt% and 20 wt%, preferably between 1×10⁻⁶ wt% and 10 wt%, preferably between 1×10⁻⁵ wt% and 5 wt%, based on the total weight of the composition, depending on the destination of the composition and the more or less pronounced desired effect.

The concentration of the additional peptide, if present, in the composition for use of the invention, ranges between 1×10⁻⁷ wt% and 20 wt%, preferably between 1×10⁻⁶ wt% and 10 wt%, preferably between 1×10⁻⁵ wt% and 5 wt%, based on the total weight of the composition.

### Method of hair repigmenting

An aspect of the invention is a method of hair repigmenting, which comprises topically applying to the skin of a subject in need thereof of an effective amount of the MIA inhibitor peptide of the invention, or a composition comprising said MIA inhibitor peptide.

"Topical application" or "topical use" means an application that is intended to act where it is applied, e.g., skin, or mucous membranes.

The "effective" amount depends on various factors, such as the age, the condition of the user, the severity of the hair greying, and the administration mode. An effective amount means a non-toxic amount enough to achieve the desired effect.

In an embodiment of the method of hair repigmenting, the MIA inhibitor peptide is combined with another peptide selected from peptide PP, SEQ ID NO: 50, and SEQ ID NO: 51.

In an embodiment, the MIA inhibitor peptide is combined with peptide PP.

In another embodiment, the MIA inhibitor peptide is combined with peptide SEQ ID NO:50.

In another embodiment, the MIA inhibitor peptide is combined with peptide SEQ ID NO:51.

The topical application may take place to the face, body, neck, scalp, hair, eyelashes, or body hair.

In an embodiment, the method of hair repigmenting may be combined with phototherapy.

The term "phototherapy" or "light therapy" as used herein includes exposure to sunlight or to radiation of specific wavelengths, for example, UV radiation, including UVA and UVB radiation. Therefore, "phototherapy" can be performed by simple exposure to sunlight, or using sunbeds, or using a radiation emitting device, in particular, a UV emitting device.

The duration of the phototherapy is not decisive, and may range, for example, from about 30 seconds to about 2 hours daily, or on alternate days, or twice or three times weekly, among other suitable options.

The present invention may be defined according to the following embodiments:
1.- Cosmetic use of a MIA inhibitor peptide as hair repigmenting agent, characterized in that the MIA inhibitor peptide has a sequence selected from SEQ ID NO: 1 to SEQ ID NO: 49, or a derivative thereof.
2.- Cosmetic use of a MIA inhibitor peptide according to embodiment 1, characterized in that the MIA inhibitor peptide has a sequence selected from SEQ ID NOs 12-20 and 27-49, or a derivative thereof; preferably selected from SEQ ID NOs 34, 37, 39-43 and 45-47, or a derivative thereof; and more preferably selected from SEQ ID NOs 34, 38, 40 and 41, or a derivative thereof.
3.- Cosmetic use of a MIA inhibitor peptide according to embodiment 1, characterized in that the MIA inhibitor peptide has the sequence SEQ ID NO: 40, or a derivative thereof, and preferably the sequence SEQ ID NO: 40.
4.- Cosmetic use of a MIA inhibitor peptide according to any of embodiments 1 to 3, characterized in that the MIA inhibitor peptide is combined with another peptide selected from peptide PP, SEQ ID NO: 50, and SEQ ID NO:51.
5.- Cosmetic use of a MIA inhibitor peptide according to embodiment 4, characterized in that the MIA inhibitor peptide is combined with peptide PP.
6.- Cosmetic use of a MIA inhibitor peptide according to embodiment 4, characterized in that the MIA inhibitor peptide is combined with peptide SEQ ID NO:50.
7.- Cosmetic use of a MIA inhibitor peptide according to embodiment 4, characterized in that the MIA inhibitor peptide is combined with peptide SEQ ID NO:51.
8.- Cosmetic use of a MIA inhibitor according to embodiment 4, characterized in that the the N-terminus of the peptide PP includes a palmitoyl group.
9.- Cosmetic use of a MIA inhibitor according to embodiment 4, characterized in that the N-terminus of SEQ ID NO: 50 includes an acetyl group, and the C-terminus an amide group (-NH₂).
10.- Cosmetic use of a MIA inhibitor according to embodiment 4, characterized in that the N-terminus of SEQ ID NO: 51 includes a palmitoyl group and the C-terminus an amide group (-NH₂).
11.- Cosmetic use of a MIA inhibitor peptide according to any of embodiments 1 to 10, characterized in that the MIA inhibitor peptide is comprised in a composition, which comprises at least a cosmetically acceptable excipient.
12.- Cosmetic use of a MIA inhibitor peptide according to embodiment 11, characterized in that the composition is for topical administration.
13.- Cosmetic use of a MIA inhibitor peptide according to embodiment 12, characterized in that the composition is for topical administration over the face, body, neck, scalp, hair, eyelashes, or body hair.
14.- Cosmetic use of a MIA inhibitor peptide according to embodiment 12 or 13, characterized in that the composition is in the form of cream, gel, cremigel, lotion, paste, foam, solution, suspension, emulsion, milk, or stick preparation.
15.- Cosmetic use of a MIA inhibitor peptide according to embodiment 14, characterized in that the composition is in the form of a lotion.
16.- Cosmetic use of a MIA inhibitor peptide according to embodiment 15, characterized in that the lotion comprises a cosmetic carrier selected from aqueous, alcoholic or hydroalcoholic.
17.- Cosmetic use of a MIA inhibitor peptide according to any of embodiments 11 to 16, characterized in that the composition comprises a cosmetically acceptable excipient selected from emollients, humectants, penetration enhancers, preservatives, viscosity controlling agents, antioxidants, pH regulators, UV filters, chelating agents, vitamins, anti-dandruff agents, perfumes, colorants, plant extracts, skin care active ingredients, hair care active ingredients, and mixtures thereof.
18.- Cosmetic use of a MIA inhibitor peptide according to any of embodiments 11 to 17, characterized in that the MIA inhibitor peptide is present in an effective amount, ranging between 1×10⁻⁷ wt% and 20 wt%, preferably between 1×10⁻⁶ wt% and 10 wt%, preferably between 1×10⁻⁵ wt% and 5 wt%, based on the total weight of the composition.
19.- Cosmetic use of a MIA inhibitor peptide according to any of embodiments 11 to 18, characterized in that the concentration of the additional peptide ranges between 1×10⁻⁷ wt% and 20 wt%, preferably between 1×10⁻⁶ wt% and 10 wt%, preferably between 1×10⁻⁵ wt% and 5 wt%, based on the total weight of the composition.
20.- A method of hair repigmenting, characterized in that it comprises topically applying to the skin of a subject in need thereof of an effective amount of the MIA inhibitor peptide having a sequence selected from SEQ ID NO: 1 to SEQ ID NO: 49, or a derivative thereof; preferably a sequence selected from SEQ ID NOs 12-20 and 27-49, or a derivative thereof; more preferably selected from SEQ ID NOs 34, 37, 39-43 and 45-47, or a derivative thereof; more preferably selected from SEQ ID NOs 34, 38, 40 and 41, or a derivative thereof; more preferably the sequence SEQ ID NO: 40, or a derivative thereof, and yet more preferably the sequence SEQ ID NO: 40, or a composition comprising said MIA inhibitor peptide.
21.- The method according to embodiment 20, characterized in that MIA inhibitor peptide is combined with another peptide selected from peptide PP, SEQ ID NO: 50, and SEQ ID NO:51.
22.- The method according to embodiment 21, characterized in that MIA inhibitor peptide is combined with peptide PP.
23.- The method according to embodiment 21, characterized in that MIA inhibitor peptide is combined with peptide SEQ ID NO:50.
24.- The method according to embodiment 21, characterized in that MIA inhibitor peptide is combined with peptide SEQ ID NO:51.
25.- The method according to any one of embodiments 20 to 24, characterized in that the topical application takes place to the face, body, neck, scalp, hair, eyelashes, or body hair.
26.- The method according to any of embodiments 20 to 25, characterized in that the method is combined with phototherapy.

### Examples

### Example 1: In vitro analysis of the anti-gray hair effects in normal human epidermal melanocytes (NHEM)

Normal Human Epidermal Melanocytes (NHEM) cells were treated during 4 hours with the drug FH535 (SigmaAldrich) at 1 mM in combination with the polypeptide of the present invention (MIA inhibitor peptide), alone, or in combination with palmitolyl prolyl proline dipeptide, peptide of SEQ ID NO: 50, or peptide SEQ ID NO;51, as shown below.

The following stock solutions of the peptides were prepared:
- 1000 ppm MIA inhibitor peptide,
- 6000 ppm palmitolyl prolyl proline dipeptide
- 500 ppm MIA inhibitor peptide + 3000 ppm palmitolyl prolyl proline dipeptide
- 200ppm SEQ ID NO: 50
- 500 ppm MIA inhibitor peptide + 100 ppm SEQ ID NO: 50
- 18ppm SEQ ID NO: 51
- 500 ppm MIA inhibitor peptide + 9 ppm SEQ ID NO: 51

All peptide stock solutions were tested at a concentration of either 0.001 wt%, 0.01wt% or 0.1wt%.

Untreated cells and cells treated with FH353 were included in the assay as controls. After the incubation period, cells were collected, RNA extracted and cDNA synthesized, as disclosed, for example, in S.A. Bustin, Absolute quantification of mRNA using real-time reverse transcription polymerase chain reaction assays, J. Mol. Endocrinol., 2000, 25(2), 169-193.

Gene expression analysis of *IRF4* and *TYR* was performed using quantitative PCR (qPCR) and specific TaqMan probes, as disclosed, for example, in Bustin, op. cit.

All data were statistically analysed using One-way ANOVA and Dunnet post-Hoc test. For all data, an α level of 5% or less (p < 0.05) was accepted to declare statistical significance.

Table I, II and III show the results for all products and combinations:

**TABLE I (stock solutions at 0.001%)**

| Product | *IRF4* | Standard error | *TYR* | Standard error |
|---|---|---|---|---|
| Control | 1.684 | ±0.141 | 1.468 | ±0.082 |
| FH535 Control | 1.033 | ±0.094 | 1.005 | ±0.037 |
| MIA inhibitor peptide (SEQ ID NO: 40) | 1.951* | ±0.291 | 1.1 | ±0.053 |
| Palmitolyl prolyl proline dipeptide (PP) | 2.434* | ±0.144 | 0.9868 | ±0.056 |
| MIA inhibitor peptide (SEQ ID NO: 40) + Palmitolyl prolyl proline dipeptide (PP) | 3.451* | ±0.293 | 1.296* | ±0.031 |

| | | | | |
|---|---|---|---|---|
| * Statistically significant | | | | |

Compound FH535 decreases the expression level of *IRF4* and *TYR* genes, which are involved in hair pigmentation.

The MIA inhibitor peptide increases the production of *IRF4* gen, as well as palmitolyl prolyl proline dipeptide.

It is observed a synergistic effect due to the combination of both peptides in the increase of production of *IRF4* and *TYR* genes, because the increase of the combination is clearly higher than the expected increase due to the separate addition of the effects of both peptides.

**TABLE II (stock solutions at 0.1%)**

| Product | *TYR* | Standard error |
|---|---|---|
| FH535 Control | 1.00 | ±0.02 |
| MIA inhibitor peptide (SEQ ID NO: 40) | 1.27* | ±0.03 |
| SEQ ID NO: 50 | 1.16 | ±0.05 |
| MIA inhibitor peptide (SEQ ID NO: 40) + SEQ ID NO: 50 | 1.34* | ±0.05 |

| | | |
|---|---|---|
| * Statistically significant | | |

It is observed a synergistic effect due to the combination of both peptides in the increase of production of *TYR* gene, because the increase of the combination is clearly higher than the expected increase due to the separate addition of the effects of both peptides, as shown in Figure 5.

**TABLE III (stock solutions at 0.01%)**

| Product | *TYR* | Standard error |
|---|---|---|
| FH535 Control | 1.00 | ±0.02 |
| MIA inhibitor peptide (SEQ ID NO: 40) | 1.18* | ±0.05 |
| SEQ ID NO: 51 | 1.18* | ±0.06 |
| MIA inhibitor peptide (SEQ ID NO: 40) + SEQ ID NO: 51 | 1.32* | ±0.04 |

| | | |
|---|---|---|
| * Statistically significant | | |

It is observed a synergistic effect due to the combination of both peptides in the increase of production of *TYR* gene, because the increase of the combination is clearly higher than the expected increase due to the separate addition of the effects of both peptides, as shown in Figure 6.

### Example 2 Preparation of capillary lotions according to the invention

Capillary lotions according to the present invention can be prepared using the components listed in Table II, being the amounts expressed in wt%:

**TABLE IV**

| Component | 2.1 | 2.2 | 2.3 | 2.4 |
|---|---|---|---|---|
| MIA inhibitor (SEQ ID NO: 40) | 0.005 | 0.005 | 0.005 | 0.005 |
| Zinc pyrithione | - | 0.3 | - | - |
| Minoxidil | - | - | - | 3.7 |
| Vitamin E | 0.5 | - | 1.5 | - |
| Calcium pantothenate | 0.1 | - | - | - |
| Niacinamide | 0.1 | - | - | - |
| Glycerine | 6.5 | - | - | - |
| Hydrogenated ethoxylated castor oil (40EO) | - | - | 0.05 | - |
| Defoamer | - | - | 0.03 | 0.014 |
| Preservative | - | - | 0.1 | - |
| Ethanol | 40.5 | 30 | 4 | 20 |
| Fragrance | 0.05 | 0.3 | 0.05 | 0.1 |
| UV-B filter | 0.09 | 0.09 | 0.09 | 0.09 |
| Water | q.s. 100 | q.s. 100 | q.s. 100 | q.s. 100 |

The process for preparing the lotions involves introducing each of the components into the hydroalcoholic solution and stirring until complete dissolution is achieved.

### Example 3 Preparation of capillary lotions according to the invention

Capillary lotions according to the present invention can be prepared using the components listed in Table III, being the amounts expressed in wt%:

**TABLE V**

| Component | 3.1 | 3.2 | 3.3 | 3.4 |
|---|---|---|---|---|
| MIA inhibitor (SEQ ID NO: 40) | 0.005 | 0.005 | 0.005 | 0.005 |
| Peptide PP | 0.009 | - | - | - |
| Peptide (SEQ ID NO: 50) | - | 0.001 | - | - |
| Peptide (SEQ ID NO: 51) | - | - | 4·10⁻⁵ | 4·10⁻⁵ |
| Zinc pyrithione | - | 0.3 | - | - |
| Minoxidil | - | - | - | 3.7 |
| Vitamin E | 0. 5 | - | 1.5 | - |
| Calcium pantothenate | 0.1 | - | - | - |
| Niacinamide | 0.1 | - | - | - |
| Glycerine | 6.5 | - | - | - |
| Hydrogenated ethoxylated castor oil (40EO) | - | - | 0.05 | - |
| Defoamer | - | - | 0.03 | 0.014 |
| Preservative | - | - | 0.1 | - |
| Ethanol | 40.5 | 30 | 4 | 20 |
| Fragrance | 0.05 | 0.3 | 0.05 | 0.1 |
| UV-B filter | 0.09 | 0.09 | 0.09 | 0.09 |
| Water | q.s. 100 | q.s. 100 | q.s. 100 | q.s. 100 |

The process for preparing the lotions involves introducing each of the components into the hydroalcoholic solution and stirring until complete dissolution is achieved.

## Claims

1. Cosmetic use of a MIA inhibitor peptide as hair repigmenting agent, **characterized in that** the MIA inhibitor peptide has a sequence selected from SEQ ID NO: 1 to SEQ ID NO: 49, or a derivative thereof.

2. Cosmetic use of a MIA inhibitor peptide according to claim 1, **characterized in that** the MIA inhibitor peptide has a sequence selected from SEQ ID NOs 12-20 and 27-49, or a derivative thereof; preferably selected from SEQ ID NOs 34, 37, 39-43 and 45-47, or a derivative thereof; and more preferably selected from SEQ ID NOs 34, 38, 40 and 41, or a derivative thereof.

3. Cosmetic use of a MIA inhibitor peptide according to claim 1, **characterized in that** the MIA inhibitor peptide has the sequence SEQ ID NO: 40, or a derivative thereof, and preferably the sequence SEQ ID NO: 40.

4. Cosmetic use of a MIA inhibitor peptide according to any of claims 1 to 3, **characterized in that** the MIA inhibitor peptide is combined with another peptide selected from peptide PP, SEQ ID NO: 50, and SEQ ID NO:51.

5. Cosmetic use of a MIA inhibitor peptide according to any of claims 1 to 4, **characterized in that** the MIA inhibitor peptide is comprised in a composition, which comprises at least a cosmetically acceptable excipient.

6. Cosmetic use of a MIA inhibitor peptide according to claim 5, **characterized in that** the composition is for topical administration.

7. Cosmetic use of a MIA inhibitor peptide according to claim 6, **characterized in that** the composition is for topical administration over the face, body, neck, scalp, hair, eyelashes, or body hair.

8. Cosmetic use of a MIA inhibitor peptide according to claim 6 or 7, **characterized in that** the composition is in the form of cream, gel, cremigel, lotion, paste, foam, solution, suspension, emulsion, milk, or stick preparation.

9. Cosmetic use of a MIA inhibitor peptide according to any of claims 5 to 8, **characterized in that** the composition comprises a cosmetically acceptable excipient selected from emollients, humectants, penetration enhancers, preservatives, viscosity controlling agents, antioxidants, pH regulators, UV filters, chelating agents, vitamins, anti-dandruff agents, perfumes, colorants, plant extracts, skin care active ingredients, hair care active ingredients, and mixtures thereof.

10. Cosmetic use of a MIA inhibitor peptide according to any of claims 5 to 9, **characterized in that** the MIA inhibitor peptide is present in an effective amount, ranging between 1×10⁻⁷ wt% and 20 wt%, preferably between 1×10⁻⁶ wt% and 10 wt%, preferably between 1×10⁻⁵ wt% and 5 wt%, based on the total weight of the composition.

11. Cosmetic use of a MIA inhibitor peptide according to any of claims 5 to 10, **characterized in that** the concentration of the additional peptide ranges between 1×10⁻⁷ wt% and 20 wt%, preferably between 1×10⁻⁶ wt% and 10 wt%, preferably between 1 ×10⁻⁵ wt% and 5 wt%, based on the total weight of the composition.

12. A method of hair repigmenting, **characterized in that** it comprises topically applying to the skin of a subject in need thereof of an effective amount of the MIA inhibitor peptide having a sequence selected from SEQ ID NO: 1 to SEQ ID NO: 49, or a derivative thereof; preferably a sequence selected from SEQ ID NOs 12-20 and 27-49, or a derivative thereof; more preferably selected from SEQ ID NOs 34, 37, 39-43 and 45-47, or a derivative thereof; more preferably selected from SEQ ID NOs 34, 38, 40 and 41, or a derivative thereof; more preferably the sequence SEQ ID NO: 40, or a derivative thereof, and yet more preferably the sequence SEQ ID NO: 40, or a composition comprising said MIA inhibitor peptide.

13. The method according to claim 12, **characterized in that** MIA inhibitor peptide is combined with another peptide selected from peptide PP, SEQ ID NO: 50, and SEQ ID NO:51.

14. The method according to claim 12 or 13, **characterized in that** the topical application takes place to the face, body, neck, scalp, hair, eyelashes, or body hair.

15. The method according to any of claims 12 to 14, **characterized in that** the method is combined with phototherapy.
